# FASCICULE DE BREVET EUROPEEN

(11) **EP 0 337 907 B1**
(45) Date de publication et mention de la délivrance du brevet: **22.09.1993**
(21) Numéro de dépôt: 89430008.6
(22) Date de dépôt: 13.04.1989
(51) Int. Cl.: C12M 1/20

(54) **Boîtes de culture simultanée de micro-organismes aérobies et anaérobies**
Behälter für die gleichzeitige Kultivierung aerober und anaerober Mikroorganismen
Containers for the simultaneous culture of aerobic and anaerobic micro-organisms

(30) Priorité: 15.04.1988 FR 8805191
(43) Date de publication de la demande: 18.10.1989
(73) Titulaire: Nicoloff, Thierry, F-13400 Aubagne (FR)
(72) Inventeur: Nicoloff, Thierry, F-13400 Aubagne (FR)

(56) Documents cités:
- DE-A- 1 933 333
- DE-A- 1 958 678
- DE-C- 839 245
- FR-A- 2 264 872
- US-A- 3 597 326

## Description

La présente invention a pour objet des boîtes de culture simultanée de micro-organismes aérobies et anaérobies.

Le secteur technique de l'invention est celui de la construction des matériels pour laboratoires d'analyses biologiques.

Dans les laboratoires de recherche ou d'analyses biologiques, on utilise des boîtes de cultures contenant un milieu nutritif, par exemple de la gélose, sur lequel on cultive des micro-organismes dont on observe le développement.

Les plus connues de ces boîtes sont les boîtes de Pétri. Ces boîtes comportent, d'une part, un bac ou récipient peu profond qui contient le milieu de culture et, d'autre part, un couvercle.

On sait qu'il existe deux grandes familles de micro-organismes, d'une part, les micro-organismes aérobies qui se développent en présence d'air et, d'autre part, les micro-organismes anaérobies qui se développent dans une atmosphère inerte, généralement une atmosphère constituée de gaz carbonique.

A ce jour, on utilise deux catégories de boîtes de culture : d'une part, des boîtes destinées à des cultures aérobies, dans lesquelles le couvercle ne s'adapte pas de façon étanche sur le bac de culture et, d'autre part, des boîtes destinées à des cultures anaérobies que l'on empile dans un récipient ou jarre, dans lequel on produit un dégagement de gaz carboniques.

Dans ce cas, si l'on veut examiner la croissance d'une culture en cours, il faut ouvrir le récipient pour atteindre individuellement chaque boîte, ce qui perturbe les cultures et exige du temps.

On connaît également des boîtes destinées à des cultures anaérobies qui comportent un couvercle transparent étanche et un produit chimique qui est placé à l'intérieur de chaque boîte et qui absorbe l'oxygène ou qui dégage du gaz carbonique au contact de l'eau ou de la vapeur d'eau.

Le brevet FR. 1.545.451 (E. REHM) décrit des boîtes pour des cultures anaérobies dans lesquelles le couvercle peut obturer hermétiquement la boîte et comporte une cloison intérieure qui délimite un espace dans lequel on place des produits chimiques qui absorbent l'oxygène.

La demande de brevet FR. A. 2.264.872 (MILES Laboratories Inc.) décrit des boîtes de culture de micro-organismes anaérobies, dans lesquelles le bac comporte une chambre qui est séparée du milieu nutritif par une cloison, laquelle chambre reçoit une composition chimique destinée à dégager un gaz, notamment du gaz carbonique et communique avec la chambre principale qui contient le milieu de culture.

Il est très souvent nécessaire dans les laboratoires d'analyses médicales qui étudient des prélèvements (sang, urine, selles etc...), de rechercher la présence des deux types de micro-organismes aérobies et anaérobies, ce qui exige de faire des cultures, d'une part, dans des boîtes pour cultures aérobies et, d'autre part, dans des boîtes pour cultures anaérobies du fait qu'il n'existe pas, à ce jour, des boîtes conçues pour effectuer simultanément ces deux types de cultures.

Il en résulte que le personnel du laboratoire doit utiliser et étiqueter plusieurs boîtes pour chaque prélèvement, ce qui multiplie les manipulations, entraîne des pertes de temps et des risques d'erreur.

De plus, les boîtes doivent êtres placées dans des étuves pour les maintenir à une température déterminée pendant l'incubation et la multiplication du nombre de boîtes entraîne un accroissement du volume des étuves.

Un objectif de la présente invention est de procurer des boîtes de culture de micro-organismes qui comportent plusieurs compartiments agencés de telle façon qu'il soit possible de réaliser, dans un même boîte, toutes les cultures aérobies et anaérobies nécessaires pour l'analyse d'un prélèvement.

Un autre objectif de la présente invention est de procurer des boîtes de culture de micro-organismes qui comportent, en outre, à l'intérieur du groupe de compartiments destinés à des cultures anaérobies, un petit volume destiné à recevoir un réactif chimique, qui dégage un gaz inerte, notamment du gaz carbonique, au contact de l'humidité ambiante.

L'invention concerne des boîtes de culture de micro-organismes qui comportent, de façon connue, un bac et un couvercle en un matériau transparent, lequel bac comporte un fond et des parois latérales et contient un milieu nutritif qui est ensemencé par un échantillon à étudier, de sorte que des micro-organismes contenus dans cet échantillon se développent sur ce milieu.

Les objectifs de l'invention sont atteints au moyen de boîtes comportant un bac qui est divisé en plusieurs compartiments séparés par des cloisons partant du fond du bac et ayant des hauteurs différentes et comporte à la fois au moins un premier compartiment, non étanche à l'air, qui est destiné à des cultures de micro-organismes aérobies et au moins un deuxième compartiment étanche à l'air, qui est destiné à des cultures de micro-organismes anaérobies.

Selon un mode de réalisation préférentiel, les compartiments non étanches sont délimités par des cloisons dont la hauteur est inférieure à celle desdites parois latérales et le groupe desdits compartiments étanches est entouré par des tronçons de parois latérales et par des cloisons ayant la même hauteur que lesdites parois et le couvercle comporte des nervures parallèles en relief, qui délimitent entre elles ou avec les bords latéraux du couvercle des rainures, dans lesquelles s'emboîtent, de façon étanche, lesdits tronçons de parois latérales et lesdites cloisons de même hauteur qui entourent le groupe de compartiment destinés à des cultures de micro-organismes anaérobies.

Selon un mode de réalisation préférentiel, une boîte selon l'invention est de forme rectangulaire, elle est divisée en compartiments rectangulaires par des cloisons parallèle à ses côtés. Chaque compartiment peut être subdivisé en deux par une cloison diagonale.

L'un au moins des compartiments rectangulaires qui est destiné à des cultures anaérobies, est délimité sur deux côtés par deux cloisons dont la hauteur est égale à celle des parois latérales qui constituent les deux autres côtés et le couvercle comporte des paires de nervures parallèles en relief, qui délimitent entre elles ou avec les parois latérales, quatre rainures dans lesquelles les bords supérieurs des cloisons et des tronçons de parois, qui délimitent le groupe de compartiments destinés à des cultures anaérobies, s'emboîtent de façon étanche.

L'invention a pour résultat des nouvelles boîtes de culture de micro-organismes qui comportent, à la fois, des compartiments pour cultures aérobies et des compartiments pour cultures anaérobies.

Les boîtes selon l'invention présentent l'avantage qu'il est possible de regrouper dans une même boîte, toutes les cultures nécessaires pour analyser biologiquement un prélèvement déterminé, ce qui permet de réduire les manipulations et d'éviter des erreurs de repérage des échantillons analysés.

Les boîtes de culture selon l'invention comportent un petit volume pouvant recevoir un réactif chimique qui dégage du gaz carbonique.

Elles permettent donc de réaliser simplement une ou plusieurs cultures anaérobies, sans avoir à placer la boîte elle-même dans une atmosphère inerte, de sorte qu'il est possible de réaliser simultanément des cultures aérobies dans les autres compartiments de la même boîte.

Les boîtes selon l'invention permettent de gagner de la place dans les étuves où elles sont placées pendant l'incubation.

Elles permettent d'observer facilement le développement des diverses cultures en cours.

La description suivante se réfère aux dessins annexés qui représentent, sans aucun caractère limitatif, un exemple de réalisation préférentiel d'une boîte de culture selon l'invention.
La figure 1 est une vue éclatée en perspective d'une boîte et de son couvercle.
La figure 2 est une coupe selon II-II de la figure 1.
La figure 3 est une vue de dessous de la face interne du couvercle.

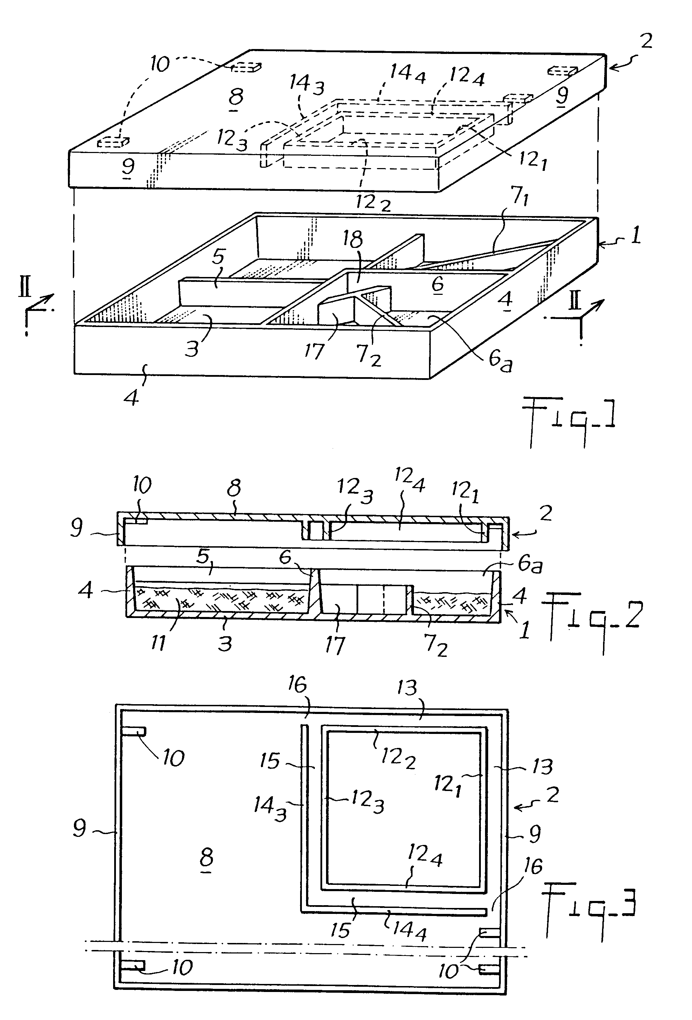

La figure 1 représente une boîte de culture de micro-organismes, qui comporte un bac 1 et un couvercle 2 en matière plastique transparente, de préférence en polystyrène.

Le bac 1 a, de préférence, une forme rectangulaire, mais il pourrait avoir d'autres formes.

Le bac 1 comporte un fond 3 et quatre parois latérales 4.

Le bac 1 est divisé en quatre compartiments rectangulaires par des cloisons internes parallèles aux parois latérales. Certaines de ces cloisons 5 ont une hauteur inférieure à celle des parois latérales 4. D'autres cloisons 6 ont une hauteur égale à celle des parois latérales.

De plus, un ou plusieurs des compartiments rectangulaires peuvent être subdivisés en deux compartiments par des cloisons diagonales 7₁, 7₂.

Les cloisons 6, dont la hauteur est égale à celle des parois latérales, en association avec des tronçons de parois latérales, entourent un groupe de compartiments 6a. Par exemple, les deux parois 6, qui encadrent un angle du bac 1 et les deux parois latérales 4 partant de cet angle, entourent un compartiment rectangulaire 6a qui est subdivisé en deux par une cloison diagonale 7₂.

Bien entendu, cette dispositif n'est qu'un exemple non limitatif et la disposition des cloisons basses 5 et des cloisons hautes 6 pourrait être différente selon la nature et le nombre de compartiments que l'on désire obtenir dans une même boîte.

Le couvercle 2 a également une forme rectangulaire. Il comporte une plaque plane 8 et quatre bords 9, perpendiculaires à celle-ci.

Les dimensions internes du couvercle sont légèrement supérieures aux dimensions externes du bac 1, de sorte que lorsque le couvercle est emboîté sur le bac, les bords 9 se trouvent à l'extérieur des parois 4, avec un faible jeu entre eux.

La figure 3 est une vue de dessus du couvercle renversé c'est-à-dire une vue de dessous de la face interne de la plaque 8.

On voit sur les figures 1 à 3 que le couvercle comporte à sa périphérie interne, plusieurs cales 10 qui sont légèrement en relief par rapport à la face interne de la plaque 9. Lorsque le couvercle est posé sur le bac 1, les bords supérieure des parois latérales 4 du bac viennent s'appuyer sur les cales 10, de sorte qu'il subsiste un faible jeu égal à l'épaisseur des cales entre lesdits bords supérieurs et la face interne du couvercle. L'air peut passer à travers ce jeu pour alimenter en oxygène les cultures ensemencées sur les milieux nutritifs 11, qui sont placés dans les compartiments délimités par les cloisons basses 5, en association avec une partie des parois latérales 4. Ces compartiments sont destinés aux cultures de micro-organismes aérobies.

On voit sur les figures 1 à 3 que le couvercle 2 comporte, dans l'angle de sa face interne qui vient se placer au-dessus du compartiment 6a, délimité par les cloisons hautes 6, quatre nervures en relief 12 qui sont parallèles aux quatre bords 9 du couvercle et qui dessinent un rectangle.

Deux des nervures 12₁ et 12₃ sont voisines de deux bords latéraux 9 et sont séparées de ceux-ci par une rainure 13, dont la largeur est légèrement supérieure à l'épaisseur des parois latérales 4.

Les deux autres nervures 12₃ et 12₄ sont doublées par deux autres nervures 14₃ et 14₄ qui leur sont parallèles et qui délimitent avec chacune d'elles une rainure 15.

Les extrémités des nervures 14₃ et 14₄ sont séparées des bords 9 du couvercle par un évidement 16 situé dans le prolongement des rainures 13.

Ainsi lorsque le couvercle 2 est emboîté sur le bac 1, les bords supérieurs des cloisons hautes 6 s'emboîtent dans les rainures 15 et les bords supérieure des tronçons de parois 4 qui complètent la périphérie du compartiment 6a délimité par les parois hautes 6, s'emboîtent dans les rainures 13.

Avantageusement les cloisons 6 et les parois 4 présentent une dépouille visible sur la figure 2, de sorte que l'emboîtement est étanche. Le groupe de compartiments 6a entouré par les cloisons hautes 6 et par des tronçons de parois qui s'emboîtent dans les rainures 15 et 13 est destiné à des cultures de micro-organismes anaérobies.

On voit sur la figure 2 que le compartiment 6a comporte, dans un de ses angles, une petite cloison 17 perpendiculaire à la cloison diagonale 7₂. Cette cloison 17 délimite, avec les deux cloisons hautes 6, un petit volume triangulaire 18 dans lequel on place un réactif chimique qui dégage un gaz inerte, par exemple du gaz carbonique, en présence de vapeur d'eau, lequel gaz crée une atmosphère inerte dans le groupe de compartiments 6a où se trouvent les cultures de micro-organismes anaérobies.

## Revendications

1. Boîte de culture de micro-organismes comportant un couvercle (2) et un bac en un matériau transparent, lequel bac comporte un fond (3) et des parois latérales (4) et contient un milieu nutritif, caractérisée en ce que ledit bec est divisé en plusieurs compartiments, au moins un premier compartiment non étanche destiné à des cultures de micro-organismes aérobies et au moins un deuxième compartiment étanche (6a) destiné à des cultures de micro-organismes anaérobies, lesquels compartiments sont séparés par au moins une cloison (6) partant du fond du bec et de même hauteur que lesdites parois (4), en ce que ledit compartiment non étanche communique avec le milieu extérieur par interposition de cales (10) situées à la périphérie interne du couvercle (2) et venant en appui sur le bord supérieur desdites parois latérales (4) et que ledit couvercle (2) comporte des rainures (13/15) dans lesquelles s'emboîtent de façon étanche, lesdites parois latérales (4) et ladite cloison (6) qui entourent ledit compartiment (6a) destiné à recevoir les cultures de micro-organismes anaérobies.

2. Boîte de culture selon le revendication 1, caractérisée en ce qu'elle comporte un premier groupe de compartiments non étanches destinés à des cultures aérobies, lesquels sont séparés par au moins une cloison (5) partant du fond du bec et dont la hauteur est inférieure à celle desdites parois latérales (4) et un deuxième groupe de compartiments étanches (6a) entourés par des tronçons desdites parois latérales (4) et par des cloisons (6) de même hauteur que lesdites parois (4), lesquelles parois (4) et cloisons (6) s'emboîtent dans lesdites rainures (13/15) du couvercle (2).

3. Boîte de culture selon l'une quelconque des revendications 1 et 2, caractérisée en ce que ledit couvercle (2) comporte des nervures parallèles en relief qui délimitent entre elles ou avec les bords latéraux (9) du couvercle, lesdites rainures (13/15).

4. Boîte de culture selon l'une quelconque des revendications 1 à 3, de forme rectangulaire, caractérisée en ce qu'elle est divisée en compartiments rectangulaires par des cloisons (5/6) parallèles à ses côtés.

5. Boîte de culture selon la revendication 4, caractérisée en ce que l'un au moins desdits compartiments rectangulaires est subdivisé en deux compartiments par une cloison diagonale (7₁/7₂).

6. Boîte de culture selon la revendication 4, caractérisée en ce qu'un au moins desdits compartiments rectangulaires (6a) est délimité sur deux côtés par deux cloisons (6), dont la hauteur est égale à celle des parois latérales (4) et est destinée à une culture de microorganismes anaérobies.

7. Boîte de culture selon la revendication 6, caractérisée en ce que l'un au moins desdits compartiments rectangulaires (6a) qui est délimité sur deux faces par deux cloisons dont la hauteur est égale à celle des parois latérales, comporte dans un de ses angles, une cloison (17), de hauteur inférieure à celle des parois laterales, qui délimite avec deux des côtés du compartiment un volume triangulaire (17), dans lequel on place un réactif chimique qui dégage un gaz au contact de l'humidité de l'atmosphère.

8. Boîte de culture selon la revendication 4, caractérisée en ce que ledit couvercle comporte, dans un des angles de sa face interne, quatre nervures en relief (12₁/12₂/12₃/12₄) parallèles aux quatre côtés du couvercle qui encadrent un rectangle, dont deux côtés (12₁/12₂) sont séparés de deux bords (9) du couvercle par une rainure (13) et dont les deux autres côtés (12₃/12₄) sont doublés par deux nervures (14₃/14₄) qui délimitent avec chacun d'eux une rainure (15).

## Claims

1. Dish for the culture of microorganisms, possessing a lid (2) and a tray made of a transparent material, which tray possesses a bottom (3) and sidewalls (4) and contains a nutrient medium, characterised in that the said tray is divided into several compartments, at least one unsealed first compartment intended for cultures of aerobic microorganisms and at least one leakproof second compartment (6a) intended for cultures of anaerobic microorganisms, which compartments are separated by at least one partition (6) rising from the bottom of the tray and of the same height as the said walls (4), in that the said unsealed compartment communicates with the external environment by the interposition of blocks (10) located at the inner periphery of the lid (2) and resting on the upper edge of the said sidewalls (4) and in that the said lid (2) possesses grooves (13/15) into which the said sidewalls (4) and the said partition (6) which surround the said compartment (6a) intended for receiving the cultures of anaerobic microorganisms fit in a leakproof manner.

2. Culture dish according to Claim 1, characterised in that it possesses a first group of unsealed compartments intended for aerobic cultures, which compartments are separated by at least one partition (5) rising from the bottom of the tray and whose height is less than that of the said sidewalls (4), and a second group of leakproof compartments (6a) surrounded by sections of the said sidewalls (4) and by partitions (6) of the same height as the said walls (4), which walls (4) and partitions (6) fit into the said grooves (13/15) of the lid (2).

3. Culture dish according to either of Claims 1 and 2, characterised in that the said lid (2) possesses raised parallel ribs which delimit, between themselves or with the side edges (9) of the lid, the said grooves (13/15).

4. Culture dish according to any one of Claims 1 to 3, of rectangular shape, characterised in that it is divided into rectangular compartments by partitions (5/6) parallel to its sides.

5. Culture dish according to Claim 4, characterised in that at least one of the said rectangular compartments is subdivided into two compartments by a diagonal partition (7₁/7₂).

6. Culture dish according to Claim 4, characterised in that at least one of the said rectangular compartments (6a) is delimited on two sides by two partitions (6) whose height is equal to that of the sidewalls (4), and is intended for a culture of anaerobic microorganisms.

7. Culture dish according to Claim 6, characterised in that at least one of the said rectangular compartments (6a) which is delimited on two faces by two partitions whose height is equal to that of the sidewalls possesses, in one of its angles, a partition (17), of height less than that of the sidewalls, which delimits with two of the sides of the compartment a triangular space (17) into which a chemical reagent which evolves a gas on contact with atmospheric moisture is placed.

8. Culture dish according to Claim 4, characterised in that the said lid possesses, in one of the angles of its inner face, four raised ribs (12₁/12₂/12₃/12₄) parallel to the four sides of the lid which enclose a rectangle, two of whose sides (12₁/12₂) are separated from two edges (9) of the lid by a groove (13), and whose other two sides (12₃/12₄) are partnered by two ribs (14₃/14₄) which delimit a groove (15) with each of them.

## Patentansprüche

1. Behälter für die Kultur von Mikroorganismen, umfassend einen Deckel (2) und eine Wanne aus durchsichtigem Material, wobei diese Wanne einen Boden (3) und Seitenwände (4) aufweist und ein Nährmedium enthält, dadurch gekennzeichnet, daß diese Wanne in mehrere Fächer aufgeteilt ist, wobei mindestens ein erstes, nicht dichtes Fach für Kulturen aerober Mikroorganismen sowie mindestens ein zweites, dichtes Fach (6a) für Kulturen anaerober Mikroorganismen vorgesehen ist und diese Fächer durch mindestens eine Trennwand (6) getrennt ist, die vom Boden der Wanne ausgeht und die gleiche Höhe wie die besagten Wände (4) aufweist, daß das undichte Fach mit der äußeren Umgebung durch das Dazwischensetzen von Keilen (10) Verbindung hat, die am inneren Umfang des Deckels (2) sitzen und auf den oberen Rand der Seitenwände (4) zum Aufliegen kommen, und daß dieser Deckel (2) Rillen (13/15) aufweist, in die sich dicht abschließend die genannten Seitenwände (4) und die genannte Trennwand (6) fügen, die das besagte Fach (6a) umschließen, welches die Kulturen anaerober Mikroorganismen aufnehmen soll.

2. Behälter für Kulturen nach Anspruch 1, dadurch gekennzeichnet, daß er eine erste Gruppe nicht dicht abschließender Fächer aufweist, die für aerobe Kulturen bestimmt sind, und die durch mindestens eine Trennwand (5) getrennt sind, die vom Boden der Wanne ausgeht und die niedriger als die genannten Seitenwände (4) ist, sowie eine zweite Gruppe dichter Fächer (6a), die von Teilstücken der genannten Seitenwände (4) und von Trennwänden (6) gleicher Höhe wie die Seitenwände (4) umgeben sind, wobei sich die Wände (4) und Trennwände (6) in die besagten Rillen (13/15) des Deckels (2) fügen.

3. Behälter für Kulturen nach einem der Ansprüche 1 und 2, dadurch gekennzeichnet, daß der besagte Deckel (2) erhabene, parallele Rippen aufweist, die untereinander oder mit den Seitenrändern (9) des Deckels die besagten Rillen (13/15) bilden.

4. Rechteckiger Behälter für Kulturen nach einem der Ansprüche 1 bis 3, dadurch gekennzeichnet, daß er durch zu seinen Seiten parallele Trennwände (5/6) in rechteckige Fächer eingeteilt ist.

5. Behälter für Kulturen nach Anspruch 4, dadurch gekennzeichnet, daß mindestens das eine der besagten, rechteckigen Fächer durch eine Diagonalwand (7₁/7₂) in zwei Fächer aufgeteilt ist.

6. Behälter für Kulturen nach Anspruch 4, dadurch gekennzeichnet, daß mindestens eines der besagten, rechteckigen Fächer (6a) an zwei Seiten durch zwei Trennwände (6) begrenzt ist, die die gleiche Höhe haben wie die Seitenwände (4), und das für die Kultur anaerober Mikroorganismen gedacht ist.

7. Behälter für Kulturen nach Anspruch 6, dadurch gekennzeichnet, daß mindestens das eine der besagten, rechteckigen Fächer (6a), das an zwei Seiten durch zwei Trennwände begrenzt ist, die die gleiche Höhe aufweisen wie die Seitenwände, in einer seiner Ecken eine Trennwand (17) aufweist, die niedriger ist als die Seitenwände und mit zwei der Seiten des Fachs einen dreieckigen Raum (18) bildet, in den ein chemisches Reagens gegeben wird, das bei Kontakt mit Luftfeuchtigkeit ein Gas abgibt.

8. Behälter für Kulturen nach Anspruch 4, dadurch gekennzeichnet, daß der besagte Deckel in einer der Ecken seiner Innenseite vier erhabene Rippen (12₁/12₂/ 12₃/12₄) aufweist, die parallel zu den vier Seiten des Deckels verlaufen, die ein Rechteck umschließen, von denen zwei Seiten (12₁/12₂) von zwei Rändern (9) des Deckels durch eine Rille (13) getrennt sind und von denen die beiden anderen Seiten (12₃/12₄) durch zwei Rippen (14₃/14₄) verstärkt sind, die mit jeder von ihnen eine Rille (15) bilden.
